# EUROPEAN PATENT APPLICATION

(11) **EP 4 582 528 A1**
(43) Date of publication of application: **09.07.2025**
(21) Application number: 23879432.5
(22) Date of filing: 04.08.2023
(51) Int. Cl.: C12M 1/00, C12N 1/00

(54) **CULTURE CONTROL DEVICE AND CULTURE CONTROL METHOD**

(30) Priority: 17.10.2022 JP 2022166507
(71) Applicant: PHC HOLDINGS CORPORATION, Tokyo 100-8403 (JP)
(72) Inventor: FUKUMOTO Satoshi, Toon-shi Ehime 791-0395 (JP); TAIRA Keisuke, Toon-shi Ehime 791-0395 (JP); TOKUMARU Tomoyoshi, Toon-shi Ehime 791-0395 (JP)
(74) Representative: Grünecker Patent- und Rechtsanwälte PartG mbB
(86) International application number: PCT/JP2023/028531
(87) International publication number: WO 2024/084779

(57) **Abstract**

A culture control device (8) includes: a calculation unit (18) that calculates a rate at which cells (10) in a culture medium (12) consume glucose, a rate at which the cells (10) produce lactic acid, and a glycolytic ratio, which is the ratio of the lactic acid production rate to the glucose consumption rate; and a selection unit (20) that selects either culture medium replacement or culture termination on the basis of the glucose consumption rate, the lactic acid production rate, and the glycolytic ratio.

## Description

### TECHNICAL FIELD

The present disclosure relates to a culture control device and a culture control method.

### BACKGROUND ART

Patent Literature 1 discloses a cell culture device that includes a cell state determination device and an operation control correction device. In this cell culture device, in order to create a database for classifying the state of the cells into normal and abnormal states and to determine the culture conditions for returning cells in an abnormal state to a normal state, culture experiments were conducted in advance under various culture conditions so as to acquire culture data.

### RELATED-ART LITERATURE

### PATENT LITERATURE

Paten Literature 1: JP 2018-117567

### SUMMARY OF INVENTION

### TECHNICAL PROBLEM

When culturing cells that cannot be secured in sufficient quantities, it is difficult to carry out various culture experiments in advance. For example, in regenerative medicine using autologous cells, the cells themselves are valuable. For this reason, it is difficult to acquire culture data of the autologous cells in advance. In general, the state of the cells varies from donor to donor. For this reason, the culture data acquired using alternative cells may not be suitable for culturing target cells. For the above reasons, the conventional culture control described above has not been easy to implement.

The present disclosure has been made in view of such a situation, and a purpose thereof is to provide a technology for easier control of culture.

### SOLUTION TO PROBLEM

The present disclosure addresses the issue described above, and one embodiment of the present disclosure relates to a culture control device. This device includes: a calculation unit that calculates a rate at which cells in a culture medium consume glucose, a rate at which the cells produce lactic acid, and a glycolytic ratio, which is the ratio of the lactic acid production rate to the glucose consumption rate; and a selection unit that selects either culture medium replacement or culture termination on the basis of the glucose consumption rate, the lactic acid production rate, and the glycolytic ratio.

Another embodiment of the present disclosure relates to a culture control method. This method includes: calculating a rate at which cells in a culture medium consume glucose, a rate at which the cells produce lactic acid, and a glycolytic ratio, which is the ratio of the lactic acid production rate to the glucose consumption rate; and selecting either culture medium replacement or culture termination on the basis of the glucose consumption rate, the lactic acid production rate, and the glycolytic ratio.

Optional combinations of the above-explained constituting elements, and implementations of the present disclosure in the form of methods, apparatuses, and systems may also be practiced as additional modes of the present disclosure.

### ADVANTAGEOUS EFFECTS OF INVENTION

According to the present disclosure, culture can be more easily controlled.

### BRIEF DESCRIPTION OF DRAWINGS

[Fig. 1] Fig. 1 is a schematic diagram of a culture system including a culture control device according to an embodiment.
[Fig. 2] Fig. 2A is a diagram showing changes over time in glucose (Glc) consumption rate and lactic acid (Lac) production rate. Fig. 2B is a diagram showing a glycolytic ratio calculated from the glucose consumption rate and the lactic acid production rate shown in Fig. 2A.
[Fig. 3] Fig. 3 is a flowchart showing an example of control executed by a culture control device.

### DESCRIPTION OF EMBODIMENTS

Hereinafter, the present disclosure will be described based on a preferred embodiment with reference to the figures. The embodiments do not limit the present disclosure and are shown for illustrative purposes, and not all the features described in the embodiments and combinations thereof are necessarily essential to the present disclosure. The same or equivalent constituting elements, members, and processes illustrated in each drawing shall be denoted by the same reference numerals, and duplicative explanations will be omitted appropriately. The scales and shapes shown in the figures are defined for convenience's sake to make the explanation easy and shall not be interpreted limitatively unless otherwise specified. Terms like "first", "second", etc., used in the specification and claims do not indicate an order or importance by any means unless specified otherwise and are used to distinguish a certain feature from the others. Those of the members that are not important in describing the embodiment are omitted from the drawings.

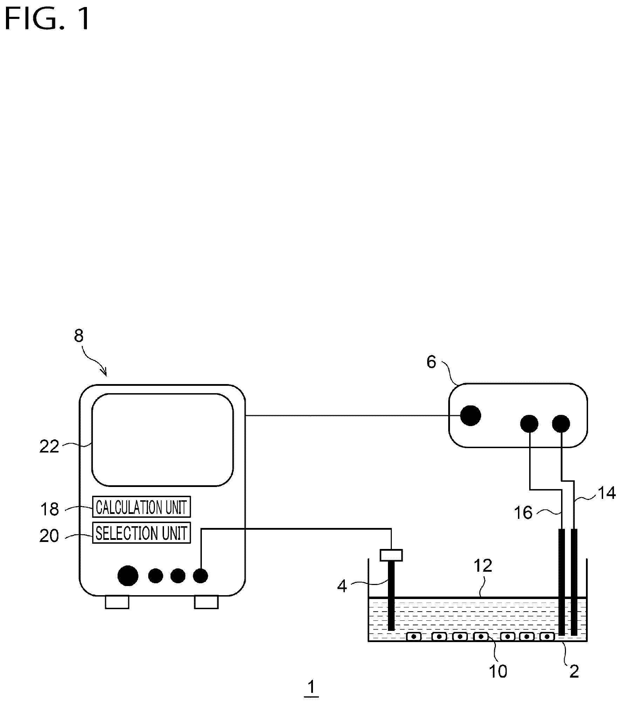

Fig. 1 is a schematic diagram of a culture system 1 including a culture control device 8 according to an embodiment. In Fig. 1, some parts of the configuration of the culture control device 8 are depicted as functional blocks. These functional blocks may be implemented by elements such as CPUs or memory of a computer or by circuits as hardware configurations, and by computer programs or the like as software configurations. Therefore, it will be obvious to those skilled in the art that the functional blocks may be implemented in a variety of manners by a combination of hardware and software.

The culture system 1 includes a culture vessel 2, a sensor 4, a culture medium replacement device 6, and a culture control device 8. The culture vessel 2 houses cells 10 to be cultured and a culture medium 12. Examples of the culture vessel 2 include known dishes, well plates, culture bags, and the like. Although not particularly limited, examples of the cells 10 include human iPS cells, for example. The culture medium 12 can be appropriately selected according to the cells 10 to be cultured.

The sensor 4 measures the glucose concentration and lactic acid concentration of the culture medium 12. An example of the sensor 4 is an electrochemical sensor provided with a working electrode, a counter electrode, and a reference electrode on a substrate. The sensor 4 has two working electrodes. One working electrode is provided with a reagent layer containing glucose oxidoreductase and a redox mediator. The other working electrode is provided with a reagent layer containing lactate oxidoreductase and a redox mediator. The sensor 4 is connected to the culture control device 8, and outputs a current corresponding to the glucose concentration and a current corresponding to the lactic acid concentration in the culture medium 12 to the culture control device 8. The structure of the sensor 4 is not limited to the above-described one. For example, a sensor for measuring glucose concentration and a sensor for measuring lactic acid concentration may be separate. Since the structure of the sensor 4 is known, further detailed description will be omitted.

The culture medium replacement device 6 replaces the culture medium 12 housed in the culture vessel **2.** The culture medium replacement device 6 has a discharge tube 14 and a suction tube 16. Further, the culture medium replacement device 6 has a culture medium tank (not shown) that houses a new culture medium 12 that is supplied to the culture vessel 2 and a drainage tank (not shown) that houses an old culture medium 12 that is recovered from the culture vessel **2.** One end of the discharge tube 14 is connected to the culture medium tank, and the other end is connected to the culture vessel **2.** One end of the suction tube 16 is connected to the drainage tank, and the other end is connected to the culture vessel **2.** The culture medium replacement device 6 performs culture medium replacement upon receiving a signal instructing culture medium replacement from the culture control device **8.** More specifically, the culture medium replacement device 6 drives a built-in pump (not shown) to supply a new culture medium 12 from the culture tank to the culture vessel 2 via the discharge tube 14, and collects the old culture medium 12 from the culture vessel 2 to the drainage tank via the suction tube 16.

The culture control device 8 controls cell culture according to the state of the cells 10 and the culture environment. As an example, the culture control device 8 includes a calculation unit 18 and a selection unit 20. The calculation unit 18 and the selection unit 20 operate when an integrated circuit constituting the units executes a program stored in the memory. The calculation unit 18 calculates a rate at which the cells 10 in the culture medium 12 consume glucose (hereinafter appropriately referred to as glucose consumption rate), a rate at which the cells 10 in the culture medium 12 produce lactic acid (hereinafter appropriately referred to as lactic acid production rate), and a glycolytic ratio. The glycolytic ratio is the ratio of the lactic acid production rate to the glucose consumption rate (lactic acid production rate/glucose consumption rate). In glycolysis, which is the main metabolic pathway the cells 10 have, one glucose molecule is converted to two lactic acid molecules. The glycolytic ratio is 100% when one glucose molecule is consumed and two lactic acid molecules are produced per unit time.

The selection unit 20 selects either the replacement of the culture medium 12 and the culture termination of the cells 10 based on the glucose consumption rate, the lactic acid production rate, and the glycolytic ratio calculated by the calculation unit 18. When the state of the cells 10 changes in the process of culture or the culture environment of the cells 10 changes, at least one of the glucose consumption rate and the lactic acid production rate can change accordingly. Further, the glycolytic ratio can also change along with this change. Therefore, the glucose consumption rate, the lactic acid production rate, and the glycolytic ratio can be used as indicators to determine the timing of culture medium change and termination of culture. The glucose consumption rate, the lactic acid production rate, and the glycolytic ratio can be easily acquired during culture. Therefore, by monitoring these three parameters during cell culture, the culture can be easily controlled.

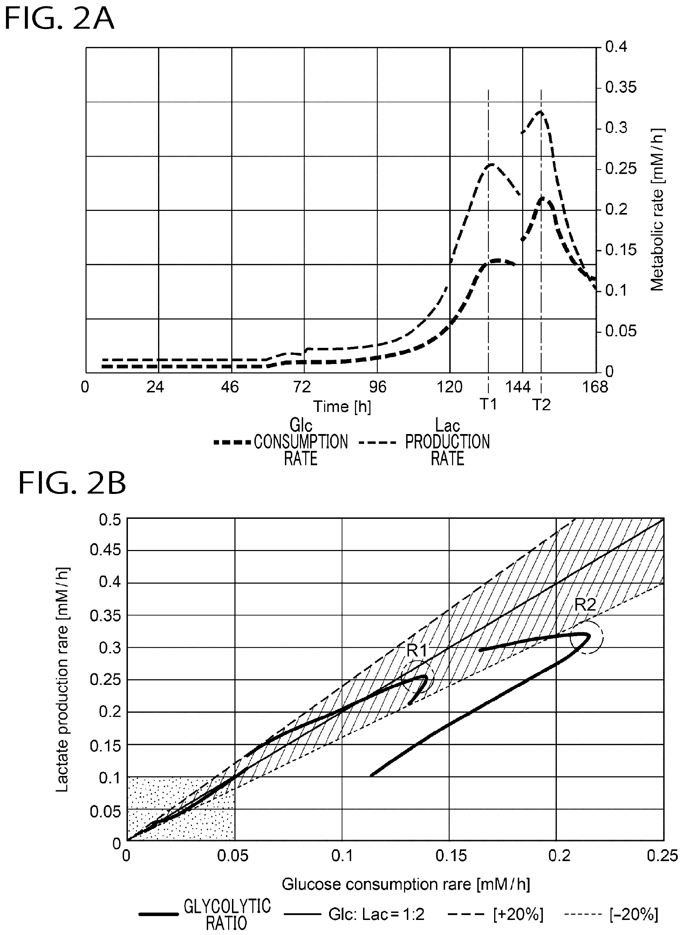

Fig. 2A is a diagram showing changes over time in glucose (Glc) consumption rate and lactic acid (Lac) production rate. Fig. 2B is a diagram showing a glycolytic ratio calculated from the glucose consumption rate and the lactic acid production rate shown in Fig. 2A. The calculation unit 18 according to the present embodiment repeatedly acquires measurement results of glucose concentration and lactic acid concentration from the sensor **4.** For example, the calculation unit 18 acquires each concentration every minute. Then, the calculation unit 18 repeatedly calculates the glucose consumption rate by differentiating the acquired glucose concentration with respect to time. Further, the acquired lactic acid concentration is differentiated with respect to time so as to repeatedly calculate the lactic acid production rate. The calculated glucose consumption rate and lactic acid production rate are stored in a memory (not shown).

In general, the cells 10 are placed in a normal culture medium 12 in substantially the same state as before the culture at the initial stage of the culture within a predetermined time from the start of the culture. As shown in Fig. 2A, in the initial stage of the culture, the glucose consumption rate and the lactic acid production rate are less than or equal to predetermined values. Further, both rates are roughly constant with only a slight increase. Also, the glycolytic ratio is constant. The calculation unit 18 calculates the initial glycolytic ratio at the initial stage of the culture. The initial glycolytic ratio serves as the criterion for selecting medium exchange or culture termination. The aforementioned "predetermined time" can be set as appropriate by the designer through experiments and simulations. As an example, the predetermined time is two hours.

The number of cells gradually increases with the passage of incubation time. This accelerates a decrease in the glucose concentration and an increase in the lactic acid concentration in the culture medium 12. Therefore, as shown in Fig. 2A, the glucose consumption rate and the lactic acid production rate gradually increase. In the example shown in Fig. 2A, both the glucose consumption rate and the lactic acid production rate peak at time T1 and then start to decrease. When the cell culture is being performed normally, the glucose consumption rate and the lactic acid production rate should increase with an increase in the number of cells. Therefore, if at least one of the glucose consumption rate and the lactic acid production rate is unchanged or decreased, it can be assumed that the culture medium 12 has deteriorated or the state of the cells 10 has changed.

Therefore, the selection unit 20 executes the selection of culture medium replacement or culture termination when at least one of the following is satisfied: the calculated glucose consumption rate is equal to or less than the previously calculated glucose consumption rate; and the calculated lactic acid production rate is equal to or less than the previously calculated lactic acid production rate.

In the selection, the selection unit 20 according to the present embodiment selects culture termination when the difference between a glycolytic ratio calculated after the calculation of the initial glycolytic ratio and the initial glycolytic ratio is equal to or greater than a predetermined threshold. Further, the selection unit 20 selects culture medium replacement when the difference is less than the threshold.

For example, the selection unit 20 converts the difference into a ratio with respect to the initial glycolytic ratio, and selects culture termination or culture medium replacement based on whether the ratio is equal to or greater than the threshold. The selection unit 20 may execute the selection based on the magnitude of the difference. The aforementioned "threshold" can be set as appropriate by the designer through experiments and simulation runs. As an example, the threshold is 20% of the initial glycolytic ratio.

When the difference between the calculated glycolytic ratio and the initial glycolytic ratio is less than the threshold, that is, when the glycolytic ratio has not changed significantly, there is a high possibility that the state of the cells 10 is maintained as the state at the initial stage of the culture. In this case, it can be assumed that the change in the glucose consumption rate and/or the change in the lactic acid production rate is caused by a deterioration of the culture environment due to a decrease in the pH of the culture medium 12, etc. Thus, the selection unit 20 selects culture medium replacement when the difference is less than the threshold. Further, when the difference is equal to or greater than the threshold, that is, when the glycolytic ratio changes significantly, there is a high possibility that the state of the cells 10 have changed from the state at the initial stage of the culture. For example, when the state of the cells 10 changes, the metabolic pathway of glucose can change from glycolysis to another. In this case, components other than lactic acid are produced from one molecule of glucose. As a result, the glycolytic ratio changes significantly. Therefore, the selection unit 20 selects the culture termination when the difference is equal to or greater than the threshold.

A chain line region R1 in Fig. 2B represents a glycolytic ratio at a time period including time T1. In the example shown in Fig. 2B, the glycolytic ratio of 100% (that is, Glc:Lac = 1:2) represents the initial glycolytic ratio, and ±20% of the initial glycolytic ratio represents a threshold for judgment. The difference in the glycolytic ratio in the time period including time T1 from the initial glycolytic ratio is less than the threshold. Therefore, the selection unit 20 selects culture medium replacement. When selecting culture medium replacement, the selection unit 20 outputs a signal instructing culture medium replacement to the culture medium replacement device **6.** Thereby, the culture of the cells 10 can be continued under a more suitable culture environment.

The culture control device 8 according to the present embodiment includes a display unit 22 that displays the selection result of the selection unit 20. When selecting the culture medium replacement, the selection unit 20 may output a signal for the notification of the execution of the culture medium replacement to the display unit 22. Thereby, the user of the culture system 1 can learn the execution of the culture medium replacement. Further, when the culture system 1 does not include a culture medium replacement device 6, the user can perform culture medium replacement at an appropriate time.

In the example shown in Fig. 2A, culture medium replacement was performed at 144 hours from the start of culture without performing culture medium replacement after the time T1 in order to observe the transition of the glucose consumption rate and the lactic acid production rate. After the continuation of cell culture after the culture medium replacement, the glucose consumption rate and the lactic acid production rate peaked again at time T2, and then began to decrease. A chain line region R2 in Fig. 2B represents a glycolytic ratio at a time period including the time T2. The difference in the glycolytic ratio in the time period including the time T2 from the initial glycolytic ratio is equal to or greater than the threshold. Therefore, the selection unit 20 selects culture termination. The selection unit 20 outputs a signal for the notification of the culture termination to the display unit 22. This allows the user of the culture system 1 to perform necessary processes at an appropriate time. Examples of the processes performed after the culture termination include subculture, differentiation induction, and disposal of the cells 10.

The selection unit 20 preferably executes the above-described selection when at least one of the following conditions is satisfied: the calculated glucose consumption rate is less than the previous glucose consumption rate; and the calculated lactic acid production rate is less than the previous lactic acid production rate. As shown in Fig. 2A, from the start of culture to a predetermined time, especially up to about 60 hours of culture, the change in the glucose consumption rate and the change in the lactic acid production rate are substantially unchanged. In these periods, there is a high possibility that the cells 10 maintain the initial state of culture, and there is a high possibility that there is less deterioration of the culture environment caused due to a decrease in the pH of the culture medium 12 or the like. Therefore, selection by the selection unit 20 is preferably not executed in these periods. For this reason, the trigger of the selection execution is limited to a decrease in the glucose consumption rate and the lactic acid production rate. Thereby, the selection unit 20 can execute selection at a more appropriate time. Therefore, culture control according to the state of the cells 10 and the culture environment can be performed with higher accuracy.

Alternatively, the selection unit 20 may execute the selection described above when at least one of the following conditions is satisfied: the calculated glucose consumption rate is equal to or greater than the predetermined value and is equal to or less than the previous glucose consumption rate; and the calculated lactic acid production rate is equal to or greater than the predetermined value and is equal to or less than the previous lactic acid production rate. As shown in Fig. 2A, near the time T1 and the time T2 when the glucose consumption rate and the lactic acid production rate turn from rise to decrease, the glucose consumption rate and the lactic acid production rate are higher than those at the initial stage of culture. For this reason, by including that the glucose consumption rate and the lactic acid production rate are equal to or greater than the respective predetermined values in the trigger of the selection execution, the selection unit 20 can execute the selection at a more appropriate time. Therefore, culture control according to the state of the cells 10 and the culture environment can be performed with higher accuracy. The aforementioned "predetermined values" can be set as appropriate by the designer through experiments and simulations. As an example, the predetermined values are 0.1 mM/h.

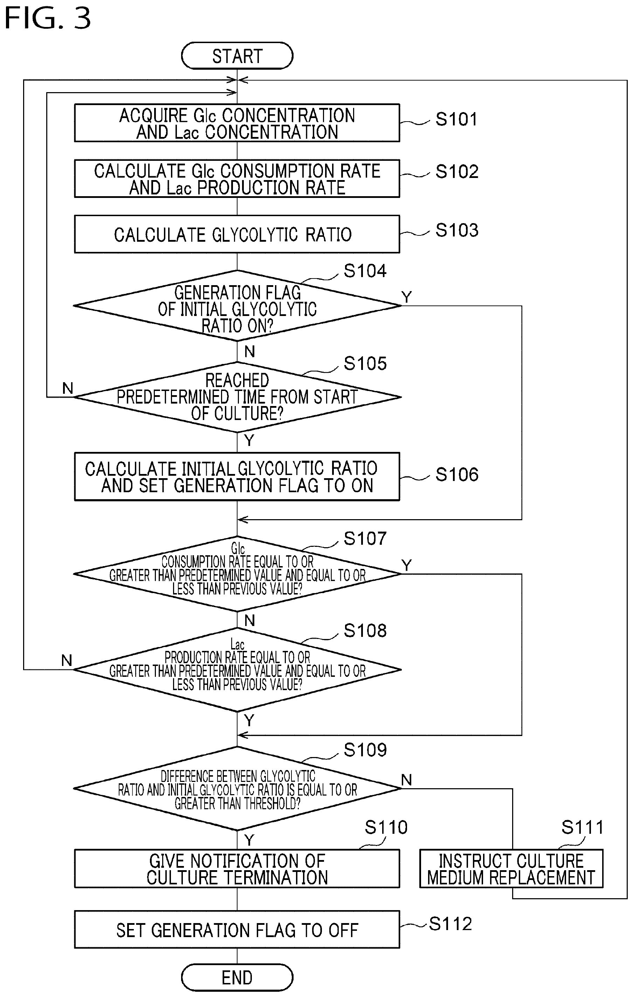

Fig. 3 is a flowchart showing an example of control executed by a culture control device 8. This flow is repeatedly executed at a predetermined time. First, the culture control device 8 acquires a glucose concentration and a lactic acid concentration from the sensor 4 (S101). Then, each acquired concentration is differentiated with respect to time to calculate the glucose consumption rate and the lactic acid production rate (S102). Further, the culture control device 8 calculates the glycolytic ratio from the calculated glucose consumption rate and the calculated lactic acid production rate (S103). Subsequently, the culture control device 8 determines whether a generation flag of the initial glycolytic ratio is on (S104).

When the generation flag is off (N in S104), the culture control device 8 determines whether the elapsed time from the start of culture has reached a predetermined time (S105). When the predetermined time is not reached (N of S105), the culture control device 8 goes back to the process in step S101. When the predetermined time is reached (Y of S105), the culture control device 8 calculates the initial glycolytic ratio and sets the generation flag to on (S106). For example, the culture control device 8 sets the average value of the glycolytic ratio calculated from the start of culture to the arrival of the predetermined time as the initial glycolytic ratio. The method for calculating the initial glycolytic ratio is not limited to those described above. For example, the culture control device 8 may define the initial glycolytic ratio as the latest glycolytic ratio held at a time when it is determined that the elapsed time from the start of culture has reached the predetermined time.

Subsequently, the culture control device 8 determines whether the last calculated (that is, the latest) glucose consumption rate is equal to or greater than the predetermined value and is equal to or less than the previously calculated glucose consumption rate (S107). When the generation flag is on (Y in S104), the culture control device 8 skips the processes in steps S105 and S106 and proceeds to the process in step S107. When the glucose consumption rate is less than the predetermined value or higher than the previous value (N of S107), the culture control device 8 determines whether the last calculated lactic acid production rate is equal to or greater than the predetermined value and is equal to or less than the previously calculated lactic acid production rate (S108).

When the lactic acid production rate is equal to or greater than the predetermined value and is equal to or less than the previous value (Y of S108), the culture control device 8 determines whether the difference between the last calculated glycolytic ratio and the initial glycolytic ratio is equal to or greater than the threshold (S109). When the glucose consumption rate is equal to or greater than the predetermined value and is equal to or less than the previous value (Y in S107), the culture control device 8 skips the process in step S108 and proceeds to the process in step S109. When the lactic acid production rate is less than the predetermined value or higher than the previous value (N in S108), the culture control device 8 goes back to the process in step S101.

When the difference between the glycolytic ratio and the initial glycolytic ratio is equal to or greater than the threshold (Y of S109), the culture control device 8 selects the culture termination. Then, the culture control device 8 outputs a signal instructing the notification of the culture termination to the display unit 22 (S110). Thereafter, the culture control device 8 sets the generation flag of the initial glycolytic ratio to off (S112) and ends the routine. When the difference between the glycolytic ratio and the initial glycolytic ratio is less than the threshold (N in S109), the culture control device 8 selects culture medium replacement. Then, the culture control device 8 outputs a signal instructing the execution of culture medium replacement to the culture medium replacement device 6 (S111), and the process returns to the process in step S101.

When returning to step S101 after the culture medium replacement is performed, the previously calculated ratio is used for the initial glycolytic ratio. In a situation where culture medium replacement is selected in the process in step S109, there is a high possibility that the state of the cells 10 is maintained. This allows for the diversion of the initial glycolytic ratio. Note that the usage mode of the initial glycolytic ratio is not limited to this structure. For example, the initial glycolytic ratio may be updated each time culture medium replacement is performed. Thereby, culture using the optimal initial glycolytic ratio for the cells 10 can be carried out more reliably. Further, the flowchart described above may be changed as follows. That is, it may be determined whether the lactic acid production rate is equal to or greater than the predetermined value and is equal to or less than the previous value in step S107, and it may be determined whether the glucose consumption rate is equal to or greater than the predetermined value and is equal to or less than the previous value in step S108. Further, in addition to the culture medium replacement according to the flowchart described above, the culture medium replacement may be performed periodically or irregularly.

As explained above, the culture control device 8 according to the present embodiment monitors the timing of culture replacement and culture termination based on the glucose consumption rate, the lactic acid production rate, and the glycolytic ratio. In other words, the principle of cell metabolism is used to determine changes in the state of the cells 10 and changes in the culture environment, and the culture is controlled according to the results. Thereby, the culture can be more easily controlled. Further, the culture control device 8 selects the culture termination when the difference between the glycolytic ratio and the initial glycolytic ratio is equal to or greater than the threshold, and selects culture medium replacement when the difference is less than the threshold. Further, the culture control device 8 executes the selection of culture termination or culture medium replacement when changes over time in at least one of the glucose consumption rate and the lactic acid production rate are unchanged or decreasing. As a result, culture according to changes in the state of the cells 10 and changes in the culture environment can be carried out more reliably.

Described above is a detailed explanation on the embodiments of the present disclosure. The above-described embodiments merely show specific examples for carrying out the present disclosure. The details of the embodiment do not limit the technical scope of the present disclosure, and many design modifications such as change, addition, deletion, etc., of the constituent elements may be made without departing from the spirit of the present invention defined in the claims. New embodiments resulting from added design change will provide the advantages of the embodiments and variations that are combined. In the above-described embodiments, the details for which such design change is possible are emphasized with the notations "according to the present embodiment", "in the present embodiment", etc. However, design change is also allowed for those without such notations. Optional combinations of the above constituting elements are also valid as embodiments of the present disclosure. Hatching applied to a cross section of a drawing does not limit the material of an object to which the hatching is applied.

The embodiments may be defined by the items described in the following.

### [Item 1]

A culture control device (8) including:
a calculation unit (18) that calculates a rate at which cells (10) in a culture medium (12) consume glucose, a rate at which the cells (10) produce lactic acid, and a glycolytic ratio, which is the ratio of the lactic acid production rate to the glucose consumption rate;
a selection unit (20) that selects either culture medium replacement or culture termination on the basis of the glucose consumption rate, the lactic acid production rate, and the glycolytic ratio.

### [Item 2]

The culture control device (8) according to Item **1,**
wherein the calculation unit (18) calculates an initial glycolytic ratio within a predetermined time from the start of culture of the cells (10), and
wherein the selection unit (20) selects the culture termination when the difference between the glycolytic ratio calculated after the calculation of the initial glycolytic ratio and the initial glycolytic ratio is equal to or greater than a predetermined threshold, and selects the culture medium replacement when the difference is less than the threshold.

### [Item 3]

The culture control device (8) according to Item 1 or **2,**
wherein the calculation unit (18) repeatedly acquires measurement results from a sensor (4) measuring glucose concentration of the culture medium (12) and a sensor (4) measuring lactic acid concentration of the culture medium (12), repeatedly calculating a glucose consumption rate from the acquired glucose concentration, and repeatedly calculating a lactic acid production rate from the acquired lactic acid concentration, and
wherein the selection unit (20) executes the selection when at least one of the following conditions is satisfied; the calculated glucose consumption rate is equal to or less than the previous glucose consumption rate; and the calculated lactic acid production rate is equal to or less than the previous lactic acid production rate.

### [Item 4]

The culture control device (8) according to Item **3,**
wherein the selection unit (20) executes the selection when at least one of the following conditions is satisfied: the calculated glucose consumption rate is less than the previous glucose consumption rate; and the calculated lactic acid production rate is less than the previous lactic acid production rate.

### [Item 5]

The culture control device (8) according to Item **3,**
wherein the selection unit (20) executes the selection when at least one of the following conditions is satisfied: the calculated glucose consumption rate is equal to or greater than a predetermined value and is equal to or less than the previous glucose consumption rate; and the calculated lactic acid production rate is equal to or greater than a predetermined value and is equal to or less than the previous lactic acid production rate.

### [Item 6]

The culture control device (8) according to any one of Items 1 through **5,** including:
a display unit (22) that displays a selection result of the selection unit (20).

### [Item 7]

A culture control device according to any one of Items 1 through 6,
when selecting culture medium replacement, the selection unit (20) outputs a signal instructing culture medium replacement to a culture medium replacement device (6).

### [Item 8]

A culture control method including:
calculating a rate at which cells (10) in a culture medium (12) consume glucose, a rate at which the cells (10) produce lactic acid, and a glycolytic ratio, which is the ratio of the lactic acid production rate to the glucose consumption rate; and
selecting either culture medium replacement or culture termination on the basis of the glucose consumption rate, the lactic acid production rate, and the glycolytic ratio.

### INDUSTRIAL APPLICABILITY

The present disclosure can be used for culture control devices and culture control methods.

### REFERENCE SIGNS LIST

1 culture system, 2 culture vessel, 4 sensor, 6 culture medium replacement device, 8 culture control device, 10 cell, 12 culture medium, 18 calculation unit, 20 selection unit, 22 display unit

## Claims

1. A culture control device comprising:
a calculation unit that calculates a rate at which cells in a culture medium consume glucose, a rate at which the cells produce lactic acid, and a glycolytic ratio, which is the ratio of the lactic acid production rate to the glucose consumption rate; and
a selection unit that selects either culture medium replacement or culture termination on the basis of the glucose consumption rate, the lactic acid production rate, and the glycolytic ratio.

2. The culture control device according to Claim **1,**
wherein the calculation unit calculates an initial glycolytic ratio within a predetermined time from the start of culture of the cells, and
wherein the selection unit selects the culture termination when the difference between the glycolytic ratio calculated after the calculation of the initial glycolytic ratio and the initial glycolytic ratio is equal to or greater than a predetermined threshold, and selects the culture medium replacement when the difference is less than the threshold.

3. The culture control device according to Claim 1 or **2,**
wherein the calculation unit repeatedly acquires measurement results from a sensor measuring glucose concentration of the culture medium and a sensor measuring lactic acid concentration of the culture medium, repeatedly calculating a glucose consumption rate from the acquired glucose concentration, and repeatedly calculating a lactic acid production rate from the acquired lactic acid concentration, and
wherein the selection unit executes the selection when at least one of the following conditions is satisfied; the calculated glucose consumption rate is equal to or less than the previous glucose consumption rate; and the calculated lactic acid production rate is equal to or less than the previous lactic acid production rate.

4. The culture control device according to Claim **3,**
wherein the selection unit executes the selection when at least one of the following conditions is satisfied; the calculated glucose consumption rate is less than the previous glucose consumption rate; and the calculated lactic acid production rate is less than the previous lactic acid production rate.

5. The culture control device according to Claim **3,**
wherein the selection unit executes the selection when at least one of the following conditions is satisfied; the calculated glucose consumption rate is equal to or greater than a predetermined value and is equal to or less than the previous glucose consumption rate; and the calculated lactic acid production rate is equal to or less than a predetermined value and is equal to or less than the previous lactic acid production rate.

6. The culture control device according to Claim 1 or 2, comprising:
a display unit that displays a selection result of the selection unit.

7. The culture control device according to Claim 1 or 2,
wherein when selecting the culture medium replacement, the selection unit outputs a signal instructing the culture medium replacement to the culture medium replacement device.

8. A culture control method comprising:
calculating a rate at which cells in a culture medium consume glucose, a rate at which the cells produce lactic acid, and a glycolytic ratio, which is the ratio of the lactic acid production rate to the glucose consumption rate; and
selecting either culture medium replacement or culture termination on the basis of the glucose consumption rate, the lactic acid production rate, and the glycolytic ratio.
